# EUROPEAN PATENT SPECIFICATION

(11) **EP 3 955 901 B1**
(45) Date of publication and mention of the grant of the patent: **06.05.2026**
(21) Application number: 20718679.2
(22) Date of filing: 17.04.2020
(51) Int. Cl.: A61K 9/70, A61K 31/135, A61P 25/24, A61P 29/00

(54) **TRANSDERMAL THERAPEUTIC SYSTEM**
TRANSDERMALES THERAPEUTISCHES SYSTEM
SYSTÈME THÉRAPEUTIQUE TRANSDERMIQUE

(30) Priority: 17.04.2019 US 201916386458; 17.04.2019 EP 19169726
(43) Date of publication of application: 23.02.2022
(73) Proprietor: LTS Lohmann Therapie-Systeme AG, 56626 Andernach (DE)
(72) Inventor: HAMMES, Florian, 56626 Andernach (DE); KLEUDGEN, Tobias, 56729 Ettringen (DE); TOMELERI, Anja, 56567 Neuwied (DE)
(74) Representative: Meissner Bolte Partnerschaft mbB
(86) International application number: PCT/EP2020/060903
(87) International publication number: WO 2020/212596

(56) References cited:
- WO-A1-2018/073227
- WO-A2-2009/017767
- JP-A- 2003 201 254
- US-A1- 2007 196 453
- US-A1- 2013 072 884
- US-A1- 2018 064 655
- US-A1- 2018 117 012
- US-B2- 9 370 495
- KOPSKY J D ET AL: "Analgesic effects of topical ketamine", MINERVA ANESTESIOLOGICA, vol. 81, no. 4, 22 May 2014 (2014-05-22), pages 440 - 449, XP093099082, Retrieved from the Internet <URL:https://www.minervamedica.it/en/getfreepdf/a0tydytIYTFId3Vva1JvV3p4RWtaamJ0QzIzdDd3Q2FPUWlzcWlubkJxWEpYYWlRNnlrTEZLazZtOVdjNG0yQg%3D%3D/R02Y2015N04A0440.pdf>
- RYDER S ET AL: "Comparative pharmacology of the optical isomers of ketamine in mice", EUROPEAN JOURNAL OF PHARMACOLOGY, ELSEVIER SCIENCE, NL, vol. 49, no. 1, 1 May 1978 (1978-05-01), pages 15 - 23, XP025516827, ISSN: 0014-2999, [retrieved on 19780501], DOI: 10.1016/0014-2999(78)90217-0
- KAPUR S ET AL: "NMDA receptor antagonists ketamine and PCP have direct effects on the dopamine D2 and serotonin 5-HT2 receptors-implications for models of schizophrenia", MOLECULAR PSYCHIATRY, NATURE PUBLISHING GROUP UK, LONDON, vol. 7, no. 8, 1 September 2002 (2002-09-01), pages 837 - 844, XP037787883, ISSN: 1359-4184, [retrieved on 20020917], DOI: 10.1038/SJ.MP.4001093
- HENKEL LTD: "DURO-TAK and GELVA Transdermal Pressure Sensitive Adhesives DURO-TAK and GELVA Transdermal Pressure Sensitive Adhesives PRODUCT SELECTION GUIDE", 20 November 2013 (2013-11-20), XP055141577, Retrieved from the Internet <URL:http://www.henkelna.com/us/content_data/330922_11061_LT5343_Product_selector2_Web863600.pdf> [retrieved on 20140919]

## Description

The present invention relates to a transdermal therapeutic system (TTS) comprising (S)-ketamine as active ingredient. The invention further concerns the use of such a system as drug, in particular for the use in the treatment of depression and/or pain.

In the past years, transdermal therapeutic systems have become increasingly important as dosage form for treating numerous diseases, because they have advantages over common dosage forms. Those are, for example, a precise and constant drug release, which is necessary for a constant concentration of the active ingredient in the blood plasma. Further, the first pass effect can be avoided and compliance can be increased, because the patient does not need to take tablets regularly. An advantage of transdermal therapeutic systems over other topical application systems such as ointments or creams is that they can be applied area accurate and therefore dosage accurate and that there is no risk of incidental wiping off the ointment with contamination of other regions. Further, ointments or tablets must be administered regularly, because a sustained release of the active ingredient usually cannot be achieved otherwise.

A few years ago, it was believed that the implementation of active ingredients in transdermal therapeutic system would be easily achievable, so that this application form would be available for a large number of active ingredients.

However, it turned out that this is not correct, because the molecular transport of ingredients via the skin poses a limiting factor. Thus, intense research is always required in order to provide transdermal therapeutic systems for the administration of new active ingredients.

The active ingredient ketamine is long known for the treatment of pain. Recently, it has also been discovered that ketamine is suitable for the treatment of psychological disorders, in particular of depression.

A transdermal therapeutic system provides an attractive option for the administration of ketamine.

Transdermal therapeutic systems for the administration of ketamine are known from the prior art.

For example, WO 2017/003935 A1 and WO 2018/195318 A1 disclose a TTS for the administration of ketamine, wherein a pressure sensitive adhesive is employed, which comprises free carboxyl groups as well as crystallization inhibitors. US 2007/196453 A1 discloses adhesive formulations, methods of drug delivery, and solidified layers for dermal delivery of a drug. The formulation can include a drug which is ketamine, a solvent vehicle, and a solidifying agent.

However, the TTS for the administration of ketamine known from the prior art require optimization with regard to the flux of the active ingredient and the utilization of the active ingredient contained in the matrix layer. Further it is of advantage to provide formulations in which ketamine is present in a stable form without utilizing crystallization inhibitors.

Thus, it was an object of the present invention to provide a TTS for the administration of (S)-ketamine, which has an optimal, i.e. as high flux of active ingredient as possible, especially in the first 2 to 12 hours after application, and in which the ketamine contained in the matrix layer is utilized in an optimal manner. Further, the ketamine contained in the TTS shall be present under conditions, where it is chemically and physically as stable as possible. Further, the TTS shall be simple in design and be economic in its production.

This task has surprisingly been solved by a transdermal therapeutic system according to claim 1.

Preferred embodiments are given in the dependent claims.

In the present disclosure, the expressions "comprising" or "containing" can also mean "consisting of".

The present invention concerns a transdermal therapeutic system, comprising a backing layer, which is not permeable for the active ingredient, and at least one matrix layer on one side of the backing layer, wherein the matrix layer contains at least one pressure sensitive adhesive, at least one penetration enhancer, and (S)-ketamine or a pharmaceutically acceptable salt or solvate thereof, characterized in that the at least one pressure sensitive adhesive comprises free hydroxyl groups, wherein the at least one pressure sensitive adhesive comprises an acrylic copolymer selected from 2-ethylhexyl acrylic acetate, vinyl acetate, and 2-hydroxyethyl acrylate comprising free hydroxyl groups, and wherein the at least one penetration enhancer is a mixture of levulinic acid and methyl laurate.

Generally, the person skilled in the art knows several types of transdermal therapeutic systems. There are DIR (drug-in-reservoir)-systems, comprising a backing layer, a reservoir layer, an adhesive layer and a detachable protective layer. In these systems, the pharmaceutically active ingredient is only present in the reservoir layer, but not in the adhesive layer, which contains at least one adhesive polymer.

Further, DIA (drug-in-adhesive)-systems are known, wherein a reservoir layer is omitted and the pharmaceutically active ingredient is present directly in the adhesive layer (also called matrix layer), which contains at least one adhesive polymer.

The advantages of DIA-systems over DIR-systems are among others a simpler production process and a lower risk of abuse. The lower risk of abuse is highly relevant in particular with regard to the active ingredient ketamine.

Thus, the transdermal therapeutic system according to the present invention is preferably a DIA-system. That is, the active ingredient, (S)-ketamine or a pharmaceutically acceptable salt or solvate thereof, is preferably present jointly with the at least one pressure sensitive adhesive in one and the same layer.

Such a TTS is characterized by its relatively simple design and thus by an economically advantageous production. Further, such a TTS according to the present invention has a higher flux of active ingredient compared to known TTSs comprising pressure sensitive adhesives which do not comprise free hydroxyl groups. Further, the (S)-ketamine contained in the matrix layer can be utilized in an optimal manner.

Moreover, the TTS according to the present invention has a high skin tolerance.

The term "utilized in an optimal manner" denotes that the ketamine contained in the matrix layer diffuses from the matrix layer into the skin of the patient to the widest possible extent during the application of the TTS on the patient's skin so that after application as little "unutilized" active ingredient remains in the matrix layer as possible.

The term "backing layer, which is not permeable for the active ingredient," denotes that the backing layer is essentially, preferably completely, impermeable for the active ingredient (S)**-**ketamine.

Suitable materials for the backing layer comprise materials such as polyester, e.g. polyethylene terephthalate, polybutylene terephthalate, polyethylene napthalate, polyolefines, such as polyethylene or polypropylene, ethylene-vinyl acetate, polyvinyl chloride, polyamide (Nylon) and/or polyurethane. The backing layer can also be composed of a composite material and preferably comprises an aluminum coated film and one of the above given materials.

A pressure sensitive adhesive is a polymer, which itself acts as pressure sensitive adhesive, as defined in DIN EN 923:2016-03.

As commonly known, a hydroxyl-group and a hydroxy-group, respectively is a - OH group.

Ketamine is (S)-(+)-2-(2-chlorophenyl)-2-(methylamino)cyclohexan-1-one ((S)-ketamine), (R)-(-)-2-(2-chlorophenyl)-2-(methylamino)cyclohexan-1-one ((R)-ketamine) as well as the racemate (RS)-(%)-2-(2-chlorophenyl)-2-(methylamino)cyclohexan-1-one. Comprised are also pharmaceutically acceptable salts and solvates of these compounds. Further comprised are mixtures of these compounds. A particularly preferred salt is ketamine·HCl.

The pharmaceutically active ingredient in the transdermal therapeutic system according to the present invention comprises (S)-ketamine and/or a pharmaceutically acceptable salt or solvate thereof, preferably (S)-ketamine·HCl.

The transdermal therapeutic system according to the present invention is characterized in that the at least one pressure sensitive adhesive comprises an acrylic copolymer selected from 2-ethylhexyl acrylic acetate, vinyl acetate, and 2-hydroxyethyl acrylate comprising free hydroxyl groups.

Further, the transdermal therapeutic system according to the present invention is preferably characterized in that the at least one pressure sensitive adhesive is obtained from 60 to 80 wt.-% 2-ethylhexyl acrylate, 1 to 10 wt.-% 2-hydroxyethyl acrylate and 20 to 30 wt.-% vinylacetate, preferably 65 to 70 wt.-% 2-ethylhexyl acrylate, 3 to 7 wt.-% 2-hydroxyethyl acrylate and 25 to 30 wt.-% vinylacetate, most preferably 68 wt.-% 2-ethylhexyl acrylate, 5 wt.-% 2-hydroxyethyl acrylate and 27 wt.-% vinylacetate as starting monomers.

Polymerization is preferably initiated by 0.1 to 0.5 wt.-%, preferably 0.3 wt.-% (wt.-% on monomer) azodiisobutyronitril.

Further, the transdermal therapeutic system according to the present invention is preferably characterized in that the at least one pressure sensitive comprises from 60 to 80 wt.-% 2-ethylhexyl acrylate, 1 to 10 wt.-% 2-hydroxyethyl acrylate and 20 to 30 wt.-% vinylacetate, preferably 65 to 70 wt.-% 2-ethylhexyl acrylate, 3 to 7 wt.-% 2-hydroxyethyl acrylate and 25 to 30 wt.-% vinylacetate, most preferably 68 wt.-% 2-ethylhexyl acrylate, 5 wt.-% 2-hydroxyethyl acrylate and 27 wt.-% vinylacetate as monomers.

The residual monomers in the at least one pressure sensitive are preferably less than 0.2 wt.-% 2-ethylhexyl acrylate, less than 0.2 wt.-% 2-hydroxyethyl acrylate and less than 4.0 wt.-% vinylacetate, preferably less or equal than 0.1 wt.-% 2-ethylhexyl acrylate, less or equal than 0.1 wt.-% 2-hydroxyethyl acrylate and less or equal than 4.0 wt.-% vinylacetate.

In another embodiment the transdermal therapeutic system according to the present invention is preferably characterized in that the at least one pressure sensitive adhesive is obtained from 60 to 80 wt.-% 2-ethylhexyl acrylate, 1 to 10 wt.-% 2-hydroxyethylacrylate and 15 to 30 wt.-% methylacrylate, preferably 65 to 75 wt.-% 2-ethylhexyl acrylate, 3 to 7 wt.-% 2-hydroxyethylacrylate and 20 to 25 wt.-% methylacrylate, most preferably 72 wt.-% 2-ethylhexyl acrylate, 5 wt.- % 2-hydroxyethylacrylate and 23 wt.-% methylacrylate as starting monomers.

Polymerization is preferably initiated by 0.1 to 0.5 wt.-%, preferably 0.2 wt.-% (wt.-% on monomer) azodiisobutyronitril.

In another embodiment the transdermal therapeutic system according to the present invention is preferably characterized in that the at least one pressure sensitive comprises from 60 to 80 wt.-% 2-ethylhexyl acrylate, 1 to 10 wt.-% 2-hydroxyethylacrylate and 15 to 30 wt.-% methylacrylate, preferably 65 to 75 wt.- % 2-ethylhexyl acrylate, 3 to 7 wt.-% 2-hydroxyethylacrylate and 20 to 25 wt.-% methylacrylate, most preferably 72 wt.-% 2-ethylhexyl acrylate, 5 wt.-% 2-hydroxyethylacrylate and 23 wt.-% methylacrylate as monomers.

The residual monomers in the at least one pressure sensitive are preferably less than 0.2 wt.-% 2-ethylhexyl acrylate, less than 0.02 wt.-% -hydroxyethylacrylate and less than 0.1 wt.-% methylacrylate, preferably less or equal than 0.1 wt.-% 2-ethylhexyl acrylate, less or equal than 0.01 wt.-% -hydroxyethylacrylate and less than 0.05 wt.-% methylacrylate.

Surprisingly, it has been found that the use of such copolymers in the matrix layer effects a high flux of active ingredient and the utilization of the ketamine contained in the matrix layer in an optimal manner.

Further, it has surprisingly been found that the transdermal therapeutic system according to the present invention has a good to sufficient adhesive strength, although the use of matrix polymers and in particular of acrylate polymers comprising carboxyl groups, which are renowned for a high adhesiveness, is abstained from.

Suitable pressure sensitive adhesives are known under the trade name DURO-TAK, in particular DURO-TAK 87-4287, DURO-TAK 87-2516, DURO-TAK 2287 or DURO-TAK 2510 of Henkel Germany.

Since the presence of free carboxyl groups can reduce the flux of the active ingredient and the utilization of the present active ingredient, the transdermal therapeutic system according to the present invention is preferably characterized in that the at least one pressure sensitive adhesive comprises less than 4 wt.%, preferably 1 to 3 wt.%, more preferably less than 1 % free carboxyl groups.

Since the presence of free carboxyl groups can reduce the flux of the active ingredient and the utilization of the present active ingredient, the transdermal therapeutic system according to the present invention is preferably characterized in that the at least one pressure sensitive adhesive comprises no free carboxyl groups.

Further, the transdermal therapeutic system according to the present invention is preferably characterized in that the at least one pressure sensitive adhesive comprises free hydroxyl groups has been obtained without a crosslinking agent.

A crosslinking agent is a chemical compound, which can effect a higher cohesion and a higher firmness of single layers of the therapeutic system. Such crosslinking agents commonly comprise metal chelates.

Omitting the crosslinking agent during the production of the pressure sensitive adhesive may also increase the flux of the active ingredient.

Further, the transdermal therapeutic system according to the present invention is preferably characterized in that the at least one pressure sensitive adhesive comprising free hydroxyl groups has been obtained with the use of a crosslinking agent.

A crosslinking agent is a chemical compound, which can effect a higher cohesion and a higher firmness of single layers of the therapeutic system. Such crosslinking agents commonly comprise metal chelates.

A crosslinking agent during the production of the pressure sensitive adhesive may also increase the flux of the active ingredient.

Further, the transdermal therapeutic system according to the present invention is preferably characterized in that the at least one pressure sensitive adhesive comprising free hydroxyl groups constitutes 60 to 90 wt.-%, preferably 70 to 85 wt.-%, of the weight of the entire matrix layer.

Further, the transdermal therapeutic system according to the present invention is characterized in that the matrix layer comprises one penetration enhancer, which is a mixture of levulinic acid and methyl laurate.

The at least one penetration enhancer is a compound, which stabilizes the active ingredient in dissolved form and thus provides a relatively high and over a long term stable resorption of the active ingredient via the skin. The term "penetration enhancer" thus may be replaced by the term "solubilizer".

Further, the transdermal therapeutic system according to the present invention is preferably characterized in that the at least one penetration enhancer is present in the matrix layer in an amount of 1 to 15 wt.-%, preferably 4 to 10 wt.-%, based on the weight of the matrix layer.

The application time, which is intended for the transdermal therapeutic system according to the present invention, preferably is at least 6 hours, more preferred at least 12 hours, and even more preferred at least 24 hours. The amount of active ingredient is preferably adapted to the desired application time.

Preferred is that the transdermal therapeutic system according to the present invention contains ketamine in the matrix layer in an amount of 1 to 25 wt.-%, preferably 5 to 15 wt.-%, based on the weight of the matrix layer.

The transdermal therapeutic system according to the present invention is further preferably characterized in that the matrix layer comprises at least one antioxidant.

The at least one antioxidant is a chemical compound, which prevents or reduces the oxidation of other substances, in particular of the active ingredient, and thus acts against aging of the therapeutical system. In particular, antioxidants are characterized by their effect as radical scavengers and by that they prevent oxidative decomposition of sensitive molecules, in particular of the active ingredient, effected by oxygen of the air. The at least one antioxidant is preferably selected from the group consisting of alpha-tocopherol, ascorbyl palmitate and/or dibutylhydroxytoluene.

Preferably, the transdermal therapeutic system according to the present invention contains the at least one antioxidant in the matrix layer in an amount of 0.001 to 5 wt.-%, preferably 0.01 to 2 wt.-%, based on the entire weight of the matrix layer.

Apart from the above mentioned components, the matrix layer may further comprise common additives. According to their function, these can be classified as softeners/plasticizers, tackifiers, stabilizers, carriers and/or fillers. The relevant, physiologically uncritical, substances are known to the person skilled in the art.

The softener/plasticizer may be selected from linear or branched, saturated or unsaturated alcohols having 6 to 20 carbon atoms, triglycerides and polyethylene glycols.

The tackifier may be selected from triglycerides, dipropylene glycol, resins, resin esters, terpenes and derivatives thereof, ethylene vinyl acetate adhesives, dimethylpolysiloxanes and polybutenes.

The stabilizer may be selected from tocopherol and ester derivatives thereof and ascorbic acid and ester derivatives thereof, and is more preferably selected from ascorbyl esters of fatty acids and tocopherol, and more preferably is ascorbyl palmitate or α-tocopherol.

Carriers and/or fillers such as silica gels, titanium dioxide and zinc oxide may be used in conjunction with the polymer in order to influence certain physical parameters, such as cohesion and bond strength, in the desired way.

Further, abuse deterrent agents can be added to the transdermal therapeutic system to prevent or at least reduce its abuse potential. Examples for substances that can be employed as abuse deterrent agents are bittering agents, gel forming agents, irritants, substances leading to acute gastrointestinal, cardiac or respiratory effects, substances leading to violent nausea or vomiting, substances leading to repugnant smells, substances inducing sleep, substances leading to deactivation or degradation of the active ingredient upon attempted extraction.

Further, the transdermal therapeutic system can also comprise an abuse deterrent feature that renders the active and/or the system ineffective when it is used in any other way than its intended use, i.e. transdermal application.

Further, additional active ingredients can be added to the transdermal therapeutic system either to counteract potential adverse effects of ketamine or to enhance the effects of ketamine. The additional active ingredients can be selected from the group of nonsteroidal anti-inflammatory drugs (NSAIDs, e.g. ibuprofen, ketoprofen, meloxicam, piroxicam, indomethacin), COX-2 inhibitors (e.g. celecoxib, etoricoxib), opioids (e.g. fentanyl, buprenorphine, morphine, codeine, oxycodone, hydrocodone, dihydromorphine, pethidine), MAOIs (irreversible and nonselective, e.g. phenelzine, tranylcypromine, isocarboxazid), MAOIs (reversible inhibitor of MAO-A, e.g. moclobemide), MAOIs (preferential inhibitor of MAO-B, e.g. deprenyl), tricyclic (and tetracyclic) antidepressants (e.g. clomipramine, imipramine, amitriptyline, nortriptyline, protriptyline, maprotiline, amoxapine, doxepin, desipramine, trimipramine), selective serotonin reuptake inhibitors (e.g. fluoxetine, sertraline, paroxetine, fluvoxamine, citalopram, escitalopram), selective noradrenaline reuptake inhibitors (e.g. reboxetine, atomoxetine), noradrenaline and dopamine reuptake inhibitor/releaser (e.g. bupropion), serotonin and noradrenaline reuptake inhibitors (e.g. venlafaxine, milnacipran, duloxetine), serotonin antagonists/reuptake inhibitors (e.g. nefazodone, trazodone), alpha2-adrenoceptor antagonist (e.g. mirtazapine).

Preferably, the transdermal therapeutic system according to the present invention is further characterized in that the matrix layer has an area weight of 30 to 400 g/m², preferably of 100 to 275 g/m².

Preferably, the transdermal therapeutic system according to the present invention is further characterized in that the transdermal therapeutic system comprises a detachable protective layer on that side of the matrix layer on which the backing layer is not arranged.

The detachable protective layer, which is in contact with the matrix and which is detached prior to application, comprises for example the same materials as used for the production of the backing layer, provided that they are made detachable, e.g. by a silicone treatment. Other detachable protective layers are polytetrafluoroethylene, treated paper, cellophane, polyvinyl chloride and the like.

Further, the present invention relates to a transdermal therapeutic system as described above as medicament.

Further, the present invention relates to a transdermal therapeutic system as described above for use in the treatment of major depressive disorder (MDD) (also known simply as depression).

In particular the described transdermal therapeutic systems can be used for the reduction of the suicidal risk and/or the treatment of treatment-resistant depression (TRD).

Major depressive disorder (MDD) is a mental disorder characterized by a pervasive and persistent low mood that is accompanied by low self-esteem and by a loss of interest or pleasure in normally enjoyable activities. Major depressive disorder is a disabling condition that adversely affects a person's family, work or school life, sleeping and eating habits, and general health.

Treatment-resistant depression (TRD) describes a condition that affects people with major depressive disorder (MDD) who do not respond adequately to a course of appropriate antidepressant medication within a certain time.

Further subtypes as recognized by The American Psychiatric Association's Diagnostic and Statistical Manual of Mental Disorders (DSM-5) are melancholic depression, atypical depression, catatonic depression, depression with anxious distress, depression with peri-partum onset and seasonal affective disorder.

Further, the present invention preferably relates to a transdermal therapeutic system as described above for use in the treatment of pain.

Pain is a distressing feeling often caused by intense or damaging stimuli. Pain that lasts a long time is called chronic or persistent, and pain that resolves quickly is called acute.

Nociceptive pain is caused by stimulation of sensory nerve fibers that respond to stimuli approaching or exceeding harmful intensity (nociceptors), and may be classified according to the mode of noxious stimulation. The most common categories are thermal, mechanical and chemical. Some nociceptors respond to more than one of these modalities and are consequently designated polymodal.

Nociceptive pain may be also divided into "visceral", "deep somatic" and "superficial somatic" pain.

Neuropathic pain is caused by damage or disease affecting any part of the nervous system involved in bodily feelings (the somatosensory system). Neuropathic pain may be divided into peripheral, central, or mixed (peripheral and central) neuropathic pain. Peripheral neuropathic pain is often described as "burning", "tingling", "electrical", "stabbing" or "pins and needles".

Further, the transdermal therapeutic systems of the present invention can be used in different administration schemes for example in consecutive or staggered administration.

In a consecutive administration transdermal systems are applied in intervals lasting at least 12 h to achieve in the blood plasma of an individual active ingredient concentrations.

The repeated administration is preferably carried out consecutively without delays, i.e., when the one or more TTSs according to the invention are removed at the end of an application interval, the one or more TTSs according to the invention for the following application interval are applied immediately. Preferably, the time interval at which there may be no TTSs according to the invention at all applied to the body is no more than 10 minutes, more preferably no more than 5 minutes.

In a staggered administration the transdermal systems is applied in intervals lasting at least 4 h to achieve in the blood plasma of an individual active ingredient concentrations.

The staggered administration is preferably carried out once daily, twice weekly or once weekly, i.e., when the one or more TTSs according to the invention are removed at the end of an application interval, the one or more TTSs according to the invention for the following application interval are applied considering a dose free interval of at least 18 hours.

In a preferred embodiment, all TTSs according to the invention are administered on the same skin area of the individual over the total period, i.e., a given skin area of the individual is overlaid or plastered repeatedly with TTSs according to the invention.

In another preferred embodiment, all TTSs according to the invention are administered each time on different skin areas of the individual over the total period, i.e., a given skin area of the individual is not overlaid or plastered repeatedly with TTSs according to the invention.

The present invention will be further described below using non-limiting examples.

### Examples:

### Example 1

The formulations of the S-ketamine-containing coating compositions of Examples 1a-c are summarized in Table 1 below. The formulations are based on weight percent as also indicated in Table 1.

**Table 1: (Reference Ex. 1a, 1c, 1d)**

| **Ingredient (Trade Name)** | **Ex. 1a** | | **Ex. 1b** | | **Ex. 1c** | | **Ex. 1d** | |
|---|---|---|---|---|---|---|---|---|
| | **Amt [g]** | **Solids [%]** | **Amt [g]** | **Solids [%]** | **Amt [g]** | **Solids [%]** | **Amt [g]** | **Solids [%]** |
| S-ketamine base | 1.20 | 11.90 | 1.00 | 9.97 | 1.20 | 12.02 | 1.00 | 9.96 |
| Acrylic adhesive in ethyl acetate. Solids content of 38.4 % by weight (DURO-TAK^{™} 387-4287) | 21.17 | 80.52 | 19.29 | 73.62 | - | - | - | - |
| Acrylic adhesive in ethyl acetate. Solids content of 47.5 % by weight (DURO-TAK^{™} 387-2052) | - | - | - | - | 16.95 | 80.42 | - | - |
| Acrylic adhesive in ethyl acetate. Solids content of 41.9 % by weight (DURO-TAK^{™} 387-2516) | - | - | - | - | - | - | 20.32 | 84.78 |
| Levulinic acid | 0.77 | 7.58 | 0.62 | 6.17 | 0.76 | 7.56 | 0.53 | 5.26 |
| Methyl laurate | - | - | 1.03 | 10.24 | - | - | - | - |
| Ethyl acetate | 2.09 | - | 3.15 | - | 6.11 | - | 3.22 | - |
| Total | 25.23 | 100.00 | 25.09 | 100.00 | 25.02 | 100.00 | 25.07 | 100.00 |
| Area Weight [g/m²] | 136.5 | | 132.8 | | 130.0 | | 122.8 | |
| S-ketamine content [mg/cm²] | 1.624 | | 1.324 | | 1.562 | | 1.222 | |

DURO-TAK 387-2516: Pressure sensitive adhesive on the basis of an acrylate vinyl acetate copolymer with comprising hydroxyl groups, obtained using a crosslinking agent.

DURO-TAK 387-4287: Pressure sensitive adhesive on the basis of an acrylate vinyl acetate copolymer comprising free hydroxyl groups, obtained without using a crosslinking agent.

DURO-TAK 387-2052: Pressure sensitive adhesive on the basis of an acrylate vinyl acetate copolymer comprising free carboxyl groups, obtained using a crosslinking agent.

For Examples 1a and 1b, a beaker was loaded with the S-ketamine base and with the solvent (ethyl acetate), and the levulinic acid and the methyl laurate (Example 1b). The acrylic pressure sensitive adhesive polymer DURO-TAK 387-4287 was added and the mixture was then stirred at up to 300 rpm until a homogeneous mixture was obtained (stirring time is about 60 min.).

For Example 1c, a beaker was loaded with the S-ketamine base and with the solvent (ethyl acetate), and the levulinic acid. The acrylic pressure sensitive adhesive polymer (DURO-TAK 387-2052) was added and the mixture was then stirred at up to 300 rpm until a homogeneous mixture was obtained (stirring time is about 60 min.).

For Example 1d, a beaker was loaded with the S-ketamine base and with the solvent (ethyl acetate), and the levulinic acid. The acrylic pressure sensitive adhesive polymer (DURO-TAK 387-2516) was added and the mixture was then stirred at up to 300 rpm until a homogeneous mixture was obtained (stirring time is about 60 min.).

The resulting S-ketamine-containing coating composition was coated on a polyethylene terephthalate film (siliconized, 75 µm thickness, which may function as release liner) and dried for approx. 15 min at room temperature and 15 min at 60 °C. The coating thickness gave an area weight of the matrix layer of 136.5 g/m² (Example 1a), 132.8 g/m² (Example 1b), 130.0 g/m² (Example 1c) and 122.8 g/m² (Example 1d), respectively. The dried film was laminated with a polyethylene terephthalate backing layer (23 µm thickness) to provide an S-ketamine-containing self-adhesive layer structure.

The individual systems were then punched out from the S-ketamine-containing self-adhesive layer structure. In specific embodiments a TTS as described above can be provided with a further self-adhesive layer of larger surface area, preferably with rounded corners, comprising a pressure-sensitive adhesive matrix layer which is free of active agent. This is of advantage when the TTS, on the basis of its physical properties alone, does not adhere sufficiently to the skin and/or when the S-ketamine-containing matrix layer, for the purpose of avoiding waste, has pronounced corners (square or rectangular shapes). The systems are then punched out and sealed into pouches of the primary packaging material.

### Preclinical set up for the assessment of local tolerance and for the determination of the plasma level of S-ketamine using Ex. 1a, Ex. 1b and Ex. 1d

### Preclinical set up Ex. 1a and Ex. 1d

Göttingen minipigs were used in this experiment. Test item formulations and corresponding placebo formulations were tested with a patch application time of 3.5 days (84 hours). Five (5) verum patches and 2 corresponding placebo formulation patches were tested on one animal for each test item formulation.
TTS size: 10 cm²

### Preclinical set up Ex. 1b

Göttingen minipig was used in this experiment. Test item formulation and corresponding placebo formulation was tested with a patch application time of one day (24 hours). Five (5) verum patches and 2 corresponding placebo formulation patches were tested on one animal.
TTS Size: 10 cm²

The assessment of local intolerance reactions according to DRAIZE (i.e. special emphasis on oedema, erythema or eschar formation) did not reveal any oedemas or erythemas at the application sites of any of the animals treated with the transdermal patches.

No other signs of local intolerance (e.g. discolourations or swellings) were noted for any of the animals at any of the application sites after patch removal.

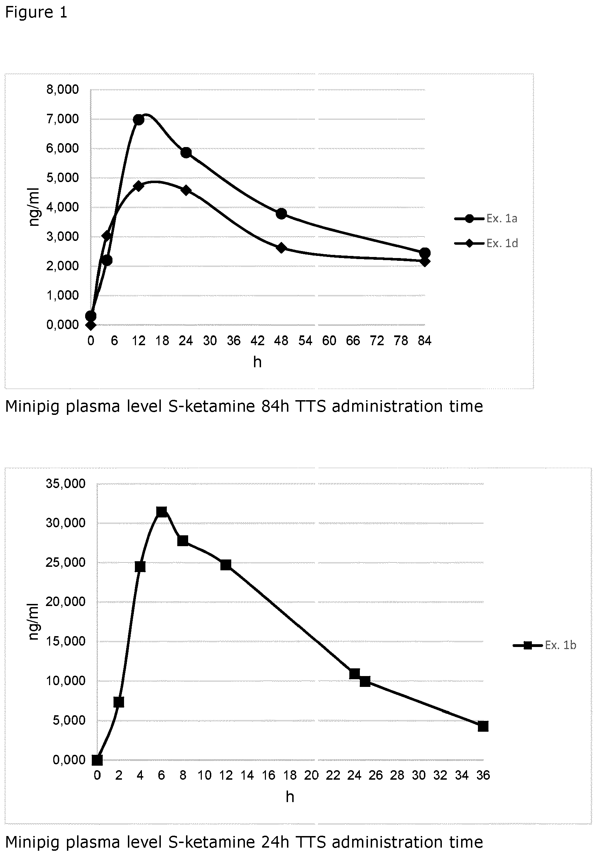

The results for the determination of the plasma level of S-ketamine are shown in Figure 1.

### Measurement of skin permeation rate

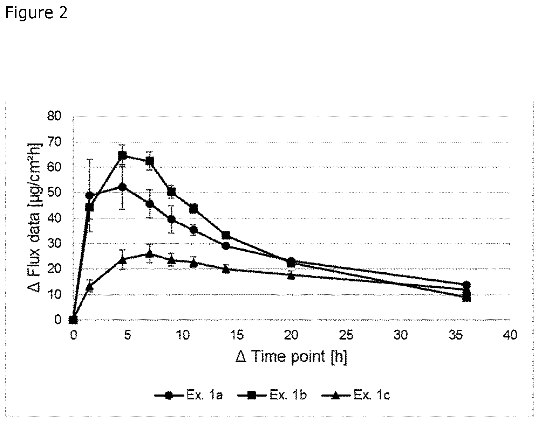

The permeated amount and the corresponding skin permeation rates of TTS prepared according to Examples 1a-1c were determined by *in vitro* experiments in accordance with the OECD Guideline (adopted April 13, 2004) and the EMA guideline on quality of transdermal patches (EMA/CHMP/QWP/608924/2014, adopted October 23, 2014), carried out with a 7.0 ml Franz diffusion cell. Split thickness human abdominal skin (female) was used. A dermatome was used to prepare skin to a thickness of 500 µm, with an intact epidermis for all TTS. Diecuts with an area of 1.152 cm² were punched from the TTS. The S-ketamine permeated amount in the receptor medium of the Franz cell (phosphate buffer solution pH 5.5 with 0.1 % saline azide as antibacteriological agent) at a temperature of 32 ± 1°C was measured and the corresponding skin permeation rate [µg/cm²*h] is calculated. The results are shown in Table 2 and Figure 2.

**Table 2**

| (Reference Ex. 1a, 1c) | | | | | | |
|---|---|---|---|---|---|---|
| **Skin permeation rate with SD [µg/(cm² h)]** | | | | | | |
| **Elapsed time [h]** | **Ex. 1a (n = 3)** | | **Ex. 1b (n = 3)** | | **Ex. 1c (n = 3)** | |
| | **Rate** | **SD** | **Rate** | **SD** | **Rate** | **SD** |
| **0** | 0 | 0 | 0 | 0 | 0 | 0 |
| **3** | 48.9 | 14.2 | 44.4 | 4.74 | 13.3 | 2.37 |
| **6** | 52.4 | 8.84 | 64.7 | 4.30 | 23.7 | 3.81 |
| **8** | 45.7 | 5.41 | 62.5 | 3.53 | 26.0 | 3.59 |
| **10** | 39.6 | 5.37 | 50.4 | 2.53 | 23.7 | 2.47 |
| **12** | 35.4 | 2.08 | 43.8 | 1.82 | 22.7 | 2.08 |
| **16** | 29.2 | 1.19 | 33.3 | 0.94 | 20.0 | 1.63 |
| **24** | 23.2 | 0.67 | 22.4 | 0.54 | 17.7 | 1.59 |
| **48** | 13.8 | 0.78 | 8.82 | 0.95 | 12.1 | 0.57 |

### Utilization of S-ketamine

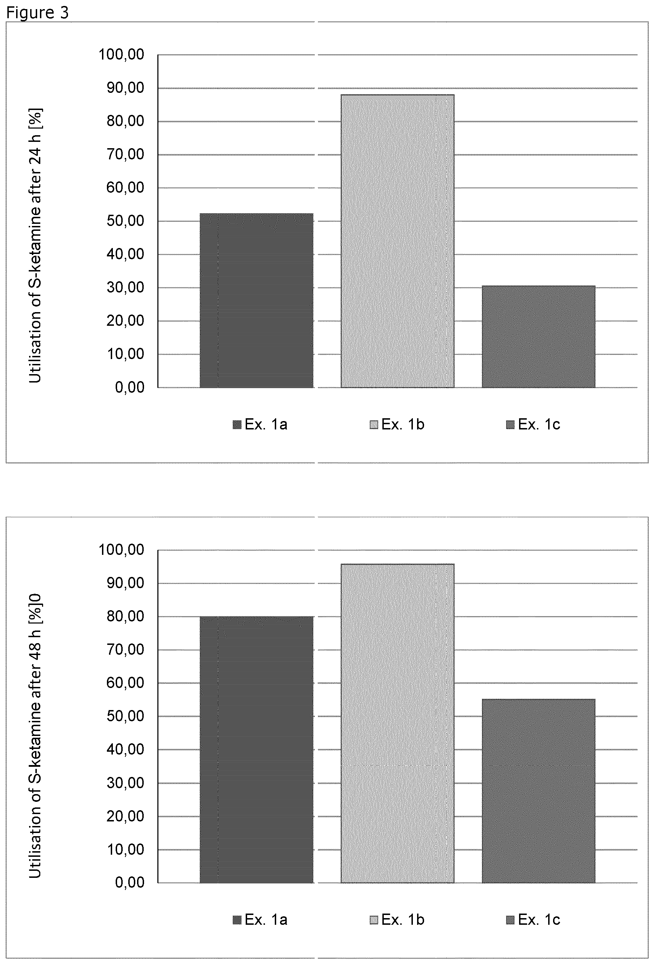

The utilization of S-ketamine at 24h and 48 h was calculated based on the residual content of the TTS after 24h and 48h and the initial S-ketamine content. The results are shown in Table 3 and Figure 3.

**Table 3:**

| (Reference Ex. 1a, 1c) | | |
|---|---|---|
| **Utilization of S-Ketamine after 24 h [%]** | | |
| Example 1a (n = 3) | Example 1b (n = 3) | Example 1c (n = 3) |
| 52.2 | 88.0 | 30.5 |

| **Utilization of S-Ketamine after 48 h [%]** | | |
|---|---|---|
| Example 1a (n = 3) | Example 1b (n = 3) | Example 1c (n = 3) |
| 79.9 | 95.7 | 55.1 |

The results summarized in Fig. 1 to Fig. 3 show an improved utilization of the active ingredient and an improved flux of active ingredient.

### Example 2

### Comparison of the skin permeation using systems according to the present invention and the prior art

The formulations of the S-ketamine-containing coating compositions of Examples 2a-c were prepared analogously as described in Example 1 and are summarized in Table 4 below. The formulations are based on weight percent as also indicated in Table 4.

**Table 4:**

| **Ingredient [wt.-%]** | Ex. 2a | Ex. 2b | Ex. 2c (Reference Example) |
|---|---|---|---|
| S-Ketamine base | 10.02 | 9.98 | 10.07 |
| DURO-TAK 87-4287 | 73.97 | 73.832 | --- |
| DURO-TAK 87-4098 | --- | --- | 54.54 |
| Methyl laurate | 10.04 | 10.10 | --- |
| Levulinic acid | 5.97 | 6.09 | 5.14 |
| Eutanol HD | --- | --- | 5.18 |
| Transcutol | --- | --- | 5.07 |
| Plastoid B | --- | --- | 20.00 |
| Area weight [g/m²] | 134.1 | 253.7 | 127.8 |

| | | | |
|---|---|---|---|
| Eutanol HD: Oleyl alcohol (enhancer) Transcutol: Diethylene glycol monoethyl ether (enhancer) Plastoid B: Copolymer of butyl methacylate and methyl methacylate | | | |

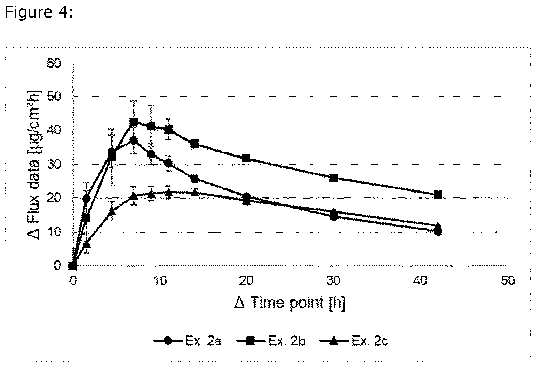

The skin permeation rate was determined analogously to Example 1 and is summarized in Figure 4. The skin permeation of the examples is according to the present invention (Ex. 2a and Ex. 2b) advantageous because the onset of the flux (the flux in the first 8 hours) is significantly higher compared to the Reference Example (Ex. 2c).

### Example 3

### Comparison of the skin permeation using systems according to the present invention with different coating weights

The formulations of the S-ketamine-containing coating compositions of Examples 3a-c were prepared analogously as described in Example 1 and are summarized in Table 5 below. The formulations are based on weight percent as also indicated in Table 5.

**Table 5**

| **Ingredient [wt.-%]** | Ex. 3a | Ex. 3b | Ex. 3c |
|---|---|---|---|
| S-Ketamine base | 10.02 | 10.01 | 10.00 |
| DURO-TAK 87-4287 | 73.97 | 74.01 | 73.89 |
| Methyl laurate | 10.04 | 9.98 | 10.10 |
| Levulinic acid | 5.97 | 6.00 | 6.01 |
| Area weight [g/m²] | 134.1 | 76.0 | 182.7 |

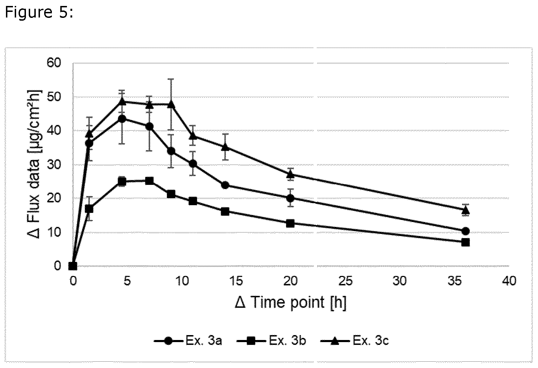

The skin permeation rate was determined analogously to Example 1 and is summarized in Figure 5. The skin permeation of the examples shows the effect of the coating weight on the onset of flux and flux profile.

### Example 4

### Comparison of the skin permeation using systems according to the present invention with cross linking agent and methyl or ethyl laurate

The formulations of the S-ketamine-containing coating compositions of Examples 4a-c were prepared analogously as described in Example 1 and are summarized in Table 6 below. The formulations are based on weight percent as also indicated in Table 6.

**Table 6 (Reference Ex. 4b)**

| **Ingredient [wt.-%]** | Ex. 4a | Ex. 4b | Ex. 4c |
|---|---|---|---|
| S-Ketamine base | 10.02 | 9.96 | 10.03 |
| DURO-TAK 87-4287 | 73.97 | 73.78 | 73.62 |
| Methyl laurate | 10.04 | --- | 9.98 |
| Ethyl laurate | --- | 10.07 | --- |
| Levulinic acid | 5.97 | 6.19 | 6.00 |
| Aluminium acetylacetonate | --- | --- | 0.37 |
| Area weight [g/m²] | 134.1 | 130.7 | 128.3 |

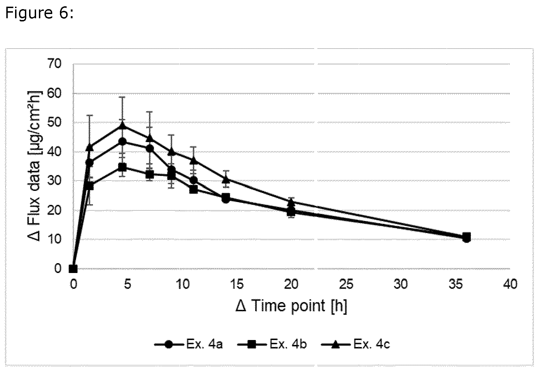

The skin permeation rate was determined analogously to Example 1 and is summarized in Figure 6. The skin permeation of the examples shows that the cross linking agent Aluminium acetylacetonate does not influence the onset of flux and that methyl laurate is more advantageous than ethyl laurate regarding the onset of flux.

### Example 5

### Comparison of the skin permeation using systems with different polymers

The formulations of the S-ketamine-containing coating compositions of Examples 5a-f are summarized in Table 7 below. The formulations are based on weight percent as also indicated in Table 7.

**Table 7**

| **Ingredient [wt.-%]** | Ex. 5a | Ex. 5b | Ex. 5c | Ex. 5d | Ex. 5e | Ex. 5f |
|---|---|---|---|---|---|---|
| S-Ketamine base | 5.00 | 5.00 | 5.00 | 5.02 | 5.00 | 5.00 |
| DURO-TAK 87-4098 | 95.00 | | | | | |
| DURO-TAK 87-9301 | | 95.00 | | | | |
| DURO-TAK 87-4287 | | | 95.00 | | | |
| DURO-TAK 87-2054 | | | | 94.98 | | |
| Plastoid B | | | | | 95.00 | |
| DURO-TAK 87-6908 | | | | | | 95.00 |
| Area weight [g/m²] | 109.2 | 105.1 | 106.0 | 105.0 | 107.3 | 100.2 |

DURO-TAK 87-4098: Pressure sensitive adhesive on the basis of an acrylate vinyl acetate copolymer without any functional groups, obtained without a crosslinking agent.

DURO-TAK 87-9301: Pressure sensitive adhesive on the basis of an acrylate polymer without any functional groups, obtained without a crosslinking agent.

DURO-TAK 87-4287: Pressure sensitive adhesive on the basis of an acrylate vinyl acetate copolymer comprising free hydroxyl groups, obtained without using a crosslinking agent.

DURO-TAK 87-2054: Pressure sensitive adhesive on the basis of an acrylate vinyl acetate copolymer comprising free carboxyl groups, obtained with using a crosslinking agent.

DURO-TAK 87-6908: Pressure sensitive adhesive on the basis of an polyisobutylene polymer comprising no functional groups, obtained without using a crosslinking agent.

Plastoid B: Pressure sensitive adhesive on the basis of a butyl methacylate and methyl methacylate copolymer comprising no functional groups, obtained without using a crosslinking agent.

For Example 5a-d and 5f, a beaker was loaded with the S-ketamine base. The adhesive polymer was added and the mixture was then stirred at up to 200 rpm until a homogeneous mixture was obtained (stirring time is about 90 min.).

The resulting S-ketamine-containing coating composition was coated on a polyethylene terephthalate film (siliconized, 75 µm thickness, which may function as release liner) and dried for approx. 15 min at room temperature and 15 min at 60 °C. The coating thickness gave an area weight of the matrix layer of 109.2 g/m² (Example 5a), 105.1g/m² (Example 5b), 106.0 g/m² (Example 5c), 105.0 g/m² (Example 5d) and 100.2 g/m² (Example 5f), respectively. The dried film was laminated with a polyethylene terephthalate backing layer (23 µm thickness) to provide an S-ketamine-containing self-adhesive layer structure.

For Example 5e, a beaker was loaded with the S-ketamine base. The butyl methacrylate and methyl methacrylate copolymer solution (50%; Plastoid B) was added and the mixture was then stirred at up to 200 rpm until a homogeneous mixture was obtained (stirring time is about 90 min.).

The resulting S-ketamine-containing coating composition was coated on a polyethylene terephthalate film (siliconized, 75 µm thickness, which may function as release liner) and dried for approx. 15 min at room temperature and 15 min at 60 °C. The coating thickness gave an area weight of the matrix layer of 107.3 g/m². The dried film was laminated with a polyethylene terephthalate backing layer (23 µm thickness) to provide an S-ketamine-containing self-adhesive layer structure.

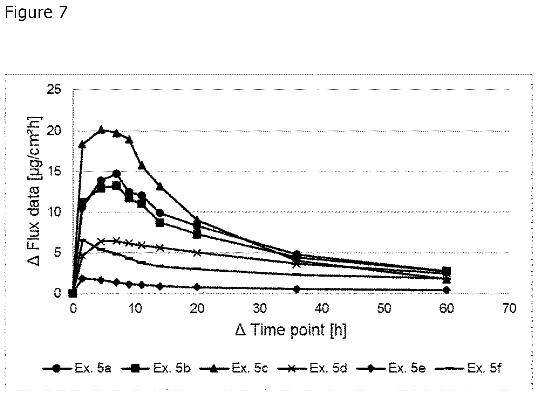

The skin permeation rate was determined analogously to Example 1 and is summarized in Figure 7. The skin permeation of the examples shows that a pressure sensitive adhesive comprising free hydroxyl groups is more advantageous regarding the skin flux rate.

### Example 6

### Comparison of the probe tack and adhesion force using systems with different polymers

The formulations of the S-ketamine-containing coating compositions of Examples 5a-d and 5f were used for the measurement of probe tack and adhesion force.

| Ex. | DURO-TAK adhesive | Ketamine free base [wt.-%] | Probe Tack [N] | RSD Probe Tack [%] | adhesion force [N] | RSD adhesion force [%] | n= |
|---|---|---|---|---|---|---|---|
| 5a | 87-4098 | 5 | 3.70 | 6.1 | 8.61 | 4.5 | 3 |
| 5b | 87-9301 | 5 | 5.13 | 2.5 | 15.50 | 2.5 | 3 |
| 5c | 87-4287 | 5 | 4.97 | 6.3 | 17.99 | 4.2 | 3 |
| 5d | 387-2054 | 5 | 5.42 | 5.8 | 17.84 | 2.5 | 3 |
| 5f | 87-6908 | 5 | 3.02 | 14.4 | 10.65 | 2.8 | 3 |

Measurement of probe tack:
Instrument: Probe Tack Tester, PT 1000 (ChemInstruments, US)
Probe Diameter: 5.0mm
Contact time of the probe with the matrix: 1sec
Sample size: 11.3cm2

Laminate strips were punched in sample size with punching tool. Afterwards the sample was mounted at the probe tack tester by using of a sample ring and the measurement was started (n=3 measurements per laminate). After each measurement, the sample ring and the probe were cleaned with gasoline (boiling range 80/110).

The average value of the 3 measurements was reported.

Measurement of adhesion force:
Instrument: Constant-rate-of-extension (CRE) tension tester, zwicki-line Z5.0 (Zwick-Roell AG, Germany)
Testing Plate: Stainless steel plates according to DIN EN 1939 / ASTM D3330/3330M-04
Sample size: width: 25mm; length: approx. 10 cm
Pre-measuring path/measuring path/post-measuring path: 5mm/50mm/5mm Testing speed: 300mm/min

The laminate was punched into 25mm wide strips with a punching tool. Afterwards die-cuts were prepared into samples of approx. 10cm length. The release liner was lifted some millimeters at the lower end to apply the elongation tape with the adhesive side to the open matrix side. Afterwards the release liner was removed completely and the sample was applied to the steel plate by hand. The measurement was started after 10 min. equilibration time and after the steel plate was fixed in the instrument, adjusted to zero and the free end of the elongation tape attached to the upper clamp. Measurement started with the parameters described above.

The average value of the 3 measurements was reported.

## Claims

1. A transdermal therapeutic system, comprising a backing layer, which is not permeable for the active ingredient, and at least one matrix layer on one side of the backing layer, wherein the matrix layer contains at least one pressure sensitive adhesive, at least one penetration enhancer, and (S)-ketamine or a pharmaceutically acceptable salt or solvate thereof, **characterized in that** the at least one pressure sensitive adhesive comprises free hydroxyl groups, wherein the at least one pressure sensitive adhesive comprises an acrylic copolymer selected from 2-ethylhexyl acrylic acetate, vinyl acetate, and 2-hydroxyethyl acrylate comprising free hydroxyl groups, and
wherein the at least one penetration enhancer is a mixture of levulinic acid and methyl laurate.

2. The transdermal therapeutic system of claim 1, **characterized in that** the at least one pressure sensitive adhesive comprises less than 4 wt.%, preferably 1-3 wt.%, more preferably less than 1 % free carboxyl groups.

3. The transdermal therapeutic system of any of the preceding claims, **characterized in that** the at least one pressure sensitive adhesive comprises no free carboxyl groups.

4. The transdermal therapeutic system of any of the preceding claims, **characterized in that** the matrix layer comprises the at least one penetration enhancer in an amount of 1 to 15 wt.-%, based on the weight of the matrix layer.

5. The transdermal therapeutic system of any of the preceding claims, **characterized in that** the matrix layer comprises ketamine in an amount of 1 to 25 wt.-%, based on the weight of the matrix layer.

6. The transdermal therapeutic system of any of the preceding claims, **characterized in that** the matrix layer comprises at least one antioxidant, preferably selected from the group consisting of alpha-tocopherol, ascorbyl palmitate and/or dibutylhydroxytoluene.

7. The transdermal therapeutic system of any of the preceding claims, **characterized in that** the matrix layer has an area weight of 30 to 400 g/m².

8. The transdermal therapeutic system of any of the preceding claims, **characterized in that** the transdermal therapeutic system comprises a detachable protective layer on that side of the matrix layer on which the backing layer is not arranged.

9. The transdermal therapeutic system of any of the preceding claims for use as a medicament.

10. The transdermal therapeutic system of any of the preceding claims for use in the treatment of depression and/or pain.

## Patentansprüche

1. Transdermales therapeutisches System, umfassend eine Rückschicht, die nicht für den Wirkstoff durchlässig ist, und wenigstens eine Matrixschicht auf einer Seite der Rückschicht, wobei die Matrixschicht wenigstens einen Haftklebstoff, wenigstens einen Permeationsverstärker und (S)-Ketamin oder ein pharmazeutisch verträgliches Salz oder Solvat davon enthält, **dadurch gekennzeichnet, dass** der wenigstens eine Haftklebstoff freie Hydroxylgruppen umfasst, wobei der wenigstens eine Haftklebstoff ein Acrylcopolymer umfasst, das aus 2-Ethylhexylacrylacetat, Vinylacetat und 2-Hydroxyethylacrylat, das freie Hydroxylgruppen umfasst, ausgewählt ist und
wobei der wenigstens eine Permeationsverstärker eine Mischung aus Lävulinsäure und Methyllaurat ist.

2. Transdermales therapeutisches System nach Anspruch 1, **dadurch gekennzeichnet, dass** der wenigstens eine Haftklebstoff weniger als 4 Gew.-%, vorzugsweise 1-3 Gew.-%, mehr bevorzugt weniger als 1 % freie Carboxylgruppen umfasst.

3. Transdermales therapeutisches System nach einem der vorstehenden Ansprüche, **dadurch gekennzeichnet, dass** der wenigstens eine Haftklebstoff keine freien Carboxylgruppen umfasst.

4. Transdermales therapeutisches System nach einem der vorstehenden Ansprüche, **dadurch gekennzeichnet, dass** die Matrixschicht den wenigstens einen Permeationsverstärker in einer Menge von 1 bis 15 Gew.-%, bezogen auf das Gewicht der Matrixschicht, umfasst.

5. Transdermales therapeutisches System nach einem der vorstehenden Ansprüche, **dadurch gekennzeichnet, dass** die Matrixschicht Ketamin in einer Menge von 1 bis 25 Gew.-%, bezogen auf das Gewicht der Matrixschicht, umfasst.

6. Transdermales therapeutisches System nach einem der vorstehenden Ansprüche, **dadurch gekennzeichnet, dass** die Matrixschicht wenigstens ein Antioxidans umfasst, vorzugsweise ausgewählt aus der Gruppe bestehend aus alpha-Tocopherol, Ascorbylpalmitat und/oder Dibutylhydroxytoluol.

7. Transdermales therapeutisches System nach einem der vorstehenden Ansprüche, **dadurch gekennzeichnet, dass** die Matrixschicht ein Flächengewicht von 30 bis 400 g/m² aufweist.

8. Transdermales therapeutisches System nach einem der vorstehenden Ansprüche, **dadurch gekennzeichnet, dass** das transdermale therapeutische System eine ablösbare Schutzschicht auf der Seite der Matrixschicht umfasst, auf der die Rückschicht nicht angeordnet ist.

9. Transdermales therapeutisches System nach einem der vorstehenden Ansprüche zur Verwendung als Medikament.

10. Transdermales therapeutisches System nach einem der vorstehenden Ansprüche zur Verwendung bei der Behandlung von Depressionen und/oder Schmerzen.

## Revendications

1. Système thérapeutique transdermique, comprenant une couche de support, qui n'est pas perméable au principe actif, et au moins une couche de matrice sur un côté de la couche de support, dans lequel la couche de matrice contient au moins un adhésif autocollant, au moins un amplificateur de pénétration, et de la (S)-kétamine ou un sel ou solvate pharmaceutiquement acceptable de celle-ci, **caractérisé en ce que** l'au moins un adhésif autocollant comprend des groupes hydroxyle libres, dans lequel l'au moins un adhésif autocollant comprend un copolymère acrylique sélectionné parmi l'acétate acrylique de 2-éthylhexyle, l'acétate de vinyle et l'acrylate de 2-hydroxyéthyle comprenant des groupes hydroxyle libres, et dans lequel l'au moins un amplificateur de pénétration est un mélange d'acide lévulinique et de laurate de méthyle.

2. Système thérapeutique transdermique selon la revendication 1,
**caractérisé en ce que** l'au moins un adhésif autocollant comprend moins de 4 % en poids, de préférence 1-3 % en poids, plus préférentiellement moins de 1 % de groupes carboxyle libres.

3. Système thérapeutique transdermique selon l'une quelconque des revendications précédentes,
**caractérisé en ce que** l'au moins un adhésif autocollant ne comprend pas de groupes carboxyle libres.

4. Système thérapeutique transdermique selon l'une quelconque des revendications précédentes,
**caractérisé en ce que** la couche de matrice comprend l'au moins un amplificateur de pénétration en une quantité de 1 à 15 % en poids, par rapport au poids de la couche de matrice.

5. Système thérapeutique transdermique selon l'une quelconque des revendications précédentes,
**caractérisé en ce que** la couche de matrice comprend de la kétamine en une quantité de 1 à 25 % en poids, par rapport au poids de la couche de matrice.

6. Système thérapeutique transdermique selon l'une quelconque des revendications précédentes,
**caractérisé en ce que** la couche de matrice comprend au moins un antioxydant, de préférence sélectionné parmi le groupe consistant en alpha-tocophérol, palmitate d'ascorbyle et/ou dibutylhydroxytoluène.

7. Système thérapeutique transdermique selon l'une quelconque des revendications précédentes,
**caractérisé en ce que** la couche de matrice présente un grammage de 30 à 400 g/m².

8. Système thérapeutique transdermique selon l'une quelconque des revendications précédentes,
**caractérisé en ce que** le système thérapeutique transdermique comprend une couche protectrice détachable du côté de la couche de matrice sur lequel n'est pas agencée la couche de support.

9. Système thérapeutique transdermique selon l'une quelconque des revendications précédentes pour une utilisation en tant que médicament.

10. Système thérapeutique transdermique selon l'une quelconque des revendications précédentes pour une utilisation dans le traitement de la dépression et/ou de la douleur.
